(19) Europäisches Patentamt
European Patent Office
Office européen des brevets

(11) Publication number: 0 031 255
B1

(12) # EUROPEAN PATENT SPECIFICATION

(45) Date of publication of patent specification: 24.07.85

(51) Int. Cl.[4]: C 01 B 33/28, B 01 J 29/28

(21) Application number: 80304659.8

(22) Date of filing: 19.12.80

(54) **Crystalline aluminosilicates, their production and use.**

(30) Priority: 21.12.79 JP 166696/79

(43) Date of publication of application:
01.07.81 Bulletin 81/26

(45) Publication of the grant of the patent:
24.07.85 Bulletin 85/30

(84) Designated Contracting States:
DE FR GB IT NL

(56) References cited:
EP-A-0 002 899
EP-A-0 022 640
US-A-3 849 463

(73) Proprietor: MITSUBISHI GAS CHEMICAL COMPANY, INC.
5-2, Marunouchi 2-chome Chiyoda-Ku
Tokyo (JP)

(72) Inventor: Suzuki, Takashi
1-4248 Tsukefune-cho
Niigata-shi Niigata-ken (JP)
Inventor: Hashimoto, Shoichiro
1741-4, Tayuhama
Niigata-shi Niigata-ken (JP)
Inventor: Nakano, Rieko
4-13-9 Higashinakajima
Niigata-shi Niigata-ken (JP)

(74) Representative: Weisert, Annekäte, Dipl.-Ing. Dr.-Ing. et al
Patentanwälte Kraus Weisert & Partner Thomas-Wimmer-Ring 15
D-8000 München 22 (DE)

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 031 255 B1

Courier Press, Leamington Spa, England.

**Description**

This invention pertains to crystalline aluminosilicates having a unique crystal form and a process for producing such crystalline aluminosilicates in the presence of fluoride ions.

Among the crystalline aluminosilicates are porous substances generally known as zeolites. They include, for example, naturally occurring zeolites such as mordenite and clinoptilonite and synthetic zeolites, A, X and Y produced by hydrothermally reacting an aqueous solution containing a silicon compound and an aluminum compound.

Recently, there were suggested synthetic zeolites having different skeletal structure from natural zeolites, such as ZSM-5 (Japanese Patent Publication No. 10,064/71), ZSM-38 (Japanese Laid-Open Patent Publication No. 120,292/77) and ZSM-34 (Japanese Laid-Open Patent Publication No. 58,499/78).

Particularly US—A—3 849 463 describes zeolites known as the ZSM-5 family having the following distinguishing X-ray diffraction peaks with the following relative intensities (VS=very strong, S=strong, M=medium, W=weak):

| d(Å) | Intensity |
|---|---|
| 11.1±0.3 | S |
| 10.0±0.3 | S |
| 5.01±0.1 | W |
| 3.85±0.1 | VS |
| 3.75±0.05 | S |
| 3.71±0.05 | S |
| 3.64±0.05 | M |

EP—A—0 022 640 describes a ZSM-5 type zeolite which can be acid-activated.

These synthetic zeolites are produced by hydrothermal reaction in the presence of organic cations. For example, ZSM-5 is produced by hydrothermal reaction in the presence of a tetrapropylammonium cation. This method can lead to the formation of a skeletal structure containing $SiO_2$ and $Al_2O_3$ in a wide range of mole ratios.

These synthetic zeolites are known to exhibit superior activity as catalysts for the isomerization and disproportionation of hydrocarbons and the dehydration reaction of oxygen-containing organic compounds.

EP—A—0 002 899 describes the olefination of methanol by a zeolite catalyst.

It is generally known however that even if the essential skeletal structure of a zeolite remains the same, its particle size has a great effect on the ease of separation of the zeolite after synthesis and the ease of the operation for preparing an ion-exchanged zeolite, and moreover when it is used as a catalyst, on the rate of reduction of its activity and the selectivity for the intended product. Particularly, zeolites are generally considered as better catalysts as their particle sizes are greater. For example, ZSM-5 synthesized by the aforesaid conventional method is a hexahedral crystal having a particle size of usually as small as about 0.1 micrometer, and from the aforesaid standpoint, there is still room for improvement in its particle size.

There have in fact been known several methods of increasing the particle size of ZSM-5, for example lowering of the pH of the starting gel (Japanese Laid-Open Patent Publication No. 134,798/78), crystallization in the presence of a sodium ion (Japanese Laid-Open Patent Publication No. 125,299) and reduction of the concentration of a tetrapropylammonium ion (Japanese Laid-Open Patent Publication No. 147,699/78). Although these methods do demonstrate some effects in increasing the particle size, they do not prove to be entirely satisfactory because crystals obtained by hydrothermal reaction vary greatly in size and shape from batch to batch or even within the same batch.

It is the object of this invention to provide a new crystalline aluminosilicate with increased particle size having a novel crystal form which as a solid acid catalyst demonstrates superior activity with a small rate of its degradation and achieves a high selectivity for the desired product.

According to the invention this object is achieved by a crystalline aluminosilicate characterized by having in its X-ray diffraction pattern determined by the standard powder method using $K_\alpha$ radiation of copper at least seven diffraction lines showing the following relative intensities at the positions of the following respective diffraction angles (2θ):

| Diffraction angle (2θ, deg) | Relative intensity |
|---|---|
| 7.9±0.3 | not more than 30 |
| 8.8±0.3 | 30—100 |
| 17.7±0.3 | 5—40 |
| 23.2±0.3 | 30—100 |
| 23.7±0.3 | 5—40 |
| 23.9±0.3 | not more than 40 |
| 24.4±0.3 | not more than 30, |

taking the intensity of the diffraction line at a diffraction angle of 8.8°±0.3° or 23.2°±0.3° as 100 and characterized in that the ratio of the length of the longest direction of the crystals thereof to that of the shortest direction of said crystals which crosses the longest direction at right angles thereto is 1.5 or higher and the length of the shortest direction is at least 14 micrometers.

The X-ray diffraction pattern is obtained by using, for example, a recording X-ray diffractometer (Geigerflex Model DS manufactured by Rigaku Denki Co., Japan) and making measurements by the standard powder diffraction method using the $K_\alpha$ radiation of copper.

The absolute value of the diffraction angles (2θ) of the several X-ray diffraction lines obtained by measuring thus may vary within a small range due to errors that might result in preparing the sample or in making the measurement. The value ±0.3 in the foregoing diffraction angles (2θ) shows the range of such a variation.

The crystalline aluminosilicate of this invention has within the diffraction angles 7—25° at least seven diffraction lines which can be clearly distinguished from each other and are characterized by having the several relative intensities indicated hereinabove. Further, there is no overlapping of these diffraction lines, i.e., two of the diffraction lines overlapping to appear as a single diffraction line, to result in the number of diffraction lines becoming less than seven.

The seven diffraction lines of the crystalline aluminosilicate of this invention can be shown typically by the following diffraction angles and relative intensities.

| Diffraction angle (2θ, deg) | Relative intensity |
|---|---|
| 7.9 | not more than 30 |
| 8.8 | 30—100 |
| 17.7 | 5—40 |
| 23.2 | 30—100 |
| 23.7 | 5—40 |
| 23.9 | not more than 40 |
| 24.4 | not more than 30 |

with the proviso that the relative intensities are shown taking the intensity of the diffraction line at a diffraction angle of 8.8° or 23.2° as 100.

The Bragg's formula can be used for obtaining the spacing (Å) of the crystal from the diffraction angle (2θ) of the X-ray diffraction line.

The relationships between the diffraction angles (2θ) and the spacings (Å) of the seven diffraction lines that are obtained by using the Bragg's formula are as follows:

| Diffraction angle (2θ, deg) | Spacing (Å) |
|---|---|
| 7.9±0.3 | 11.2±0.4 |
| 8.8±0.3 | 10.0±0.4 |
| 17.7±0.3 | 5.01±0.1 |
| 23.2±0.3 | 3.83±0.05 |
| 23.7±0.3 | 3.75±0.05 |
| 23.9±0.3 | 3.72±0.05 |
| 24.4±0.3 | 3.65±0.05 |

The crystalline aluminosilicates (abbreviated hereinafter to crystals) of this invention are characterized by having a unique crystal form in that as compared with the conventional ZSM-5 crystals there is externally a special growth at a specific crystal face only. When observed microscopically, the crystal of this invention is basically a pillar-like octahedron. Two opposing surfaces of the octahedron are hexagonal, and the other six surfaces are tetragonal. The crystal of this invention also encompasses those having the shape of a pillar-like octahedron whose vertices and edges have been worn. In the crystal of the invention, the ratio of the length of its long axis (the longest distance between opposing vertices of the hexagonal surface) to that of its short axis (the shortest of the axes which cross the long axis at right angles thereto) is 1.5 or higher. Sometimes, the length of the long axis reaches as large as several tens of micrometers. Moreover, the crystal of this invention may exist not only as a single crystal but also as a polycrystal such as a twinned crystal.

These external characteristics of the crystals of this invention bring about various advantages in performing the step of separating the crystals after their preparation and the step of preparing the catalyst.

The crystals of this invention are also distinctive in that they exhibit catalytic properties entirely different from the conventional zeolites.

In the case of the conventional crystals, the prepared crystals are calcined, ion-exchanged with ammonium chloride and calcined to convert them into so-called "$H^+$ type" crystals before they are used as an acid catalyst. On the other hand, the crystals of this invention usually do not exhibit catalytic activity when used as such. Further, their activity as an acid catalyst is not manifested by the usual activation operation, i.e. the calcination-ion exchange-calcination operation, mentioned above. The crystals of this invention, when treated with an acid such as hydrochloric acid, etc. are activated to acid catalysts.

This characteristic of the crystals of this invention makes it possible to use them as such as an adsorbent for separating the hydrocarbons. Again, they can be used as a solid acid for isomerizing the hydrocarbons by an acid treatment.

The crystals of this invention may also be regarded as being distinctive in their composition in that they are produced, as hereinafter described, in the presence of a fluorine compound. Hence, they differ from the conventional zeolites in their composition, and there can be provided crystalline aluminosilicates which genetically contain fluorine.

It has however been made known by our investigations that the presence or absence or of the amount contained of fluorine had hardly any effect on the catalytic acitivity of the crystals of this invention in using them, for example, as a solid acid catalyst. For instance, there are instances in which the content of fluorine is reduced or disappears when the crystals of this invention are calcined and acid treated. Even in such cases, there was hardly any substantial effect on the catalytic acitivity of the crystals as a solid acid catalyst.

This invention also provides a convenient process for producing the crystalline aluminosilicates. Specifically, it is a process for producing a crystalline aluminosilicate by submitting a silicon compound and an aluminum compound to a hydrothermal reaction in an aqueous medium in the copresence of an organic cation and an alkali, characterized by carrying out said hydrothermal reaction in the presence of a fluorine compound.

As the compounds other than the fluorine compound that are used in the process of this invention, i.e., the silicon compound, aluminum compound, the compound providing the organic cations, and alkali, the same compounds as those known in the prior art of producing the zeolites by the hydrohtermal synthetic method can be used.

The fluorine compound to be used in the invention process may be any of the fluorine compounds so long as it is one that forms the fluoride ions during the hydrothermal synthetic reaction. Those which form fluoride ions when dissolved in water, for example, sodium fluoride and ammonium fluoride can naturally be used. In addition, those which form the fluoride ions when submitted to hydrolysis such as silicon fluoride, boron fluoride and sulfur fluoride can also be used. Of these fluorine compounds, those which

form the fluoride ions by dissolving in water, particularly sodium fluoride and ammonium fluoride, are preferred.

Usable as the silicon compound are those compounds that have been usually used as the silica source in the hydrothermal synthetic reaction, such as sodium silicate, solid silica, silica gel and silica sol, of which preferred is silica sol. For example, conveniently used are such products as Ludox (trade mark) produced by Du Pont Company, Snowtex (trade mark) produced by Nissan Chemical Industries, Ltd., and Cataloid (trade mark) produced by Shokubai Kasei Ind., Ltd.

Example of the aluminum compound which can be used are sodium aluminate and freshly prepared aluminum hydroxide.

On the other hand, the sodium compounds are used as the alkali in the process of this invention. From practical considerations the use of caustic soda is convenient. It may be of the usually used grade. While it is also possible to use as the alkali compound those other than the sodium compounds, in such cases extreme difficulty if usually experienced in forming the crystals.

When sodium aluminate has been used as the aluminum compound, the sodium aluminate also functions as an alkali metal compound.

As a practical matter, the quaternary ammonium cation is advantageously used as the organic cation. For example, this quaternary ammonium ion is represented by the formula

$$R_4N^+ \qquad (1)$$

wherein R is a lower alkyl group. As the lower alkyl group represented by R, preferred are the lower alkyl groups of 1—4 carbon atoms such as methyl, ethyl, propyl and butyl.

As such a quaternary ammonium ion, mention can be made of such ions as tetramethylammonium, tetraethylammonium, tetrapropylammonium and tetrabutylammonium.

The quaternary ammonium ion of the foregoing general formula (1) can be derived from a quaternary ammonium compound of the formula

$$R_4NX \qquad (2)$$

wherein R is as defined in the case of formula (1), X is a halogen atom such as chlorine, bromine and iodine, or an anion such as a residue of an acid, for example sulfate ion, a hydroxy ion; or it can also be obtained by forming the foregoing quaternary ammonium compound of formula (2) in the reaction system by using a tertiary amine of the formula

$$R_3N \qquad (3)$$

wherein R is as defined in the case of formula (1), conjointly with a compound of the formula

$$RX \qquad (4)$$

wherein R and X are as defined in the case of formula (2).

Specific examples of the quaternary ammonium compounds of the general formula (2) include such compounds as tetrapropylammonium bromide, tetramethylammonium bromide, tetraethylammonium bromide, tetrapropylammonium chloride, tetrabutylammonium bromide and tetrabutylammonium chloride; as well as the tetramethylammonium-, tetraethylammonium-, tetrapropylammonium- and tetrabutylammonium-hydroxides and -sulfates.

As the tertiary amines of the general formula (3), there can be mentioned, for example, trimethylamine, triethylamine, tripropylamine and tributylamine.

Specific examples of the compounds of the general formula (4) are methyl bromide, ethyl bromide, propyl bromide, propyl chloride, butyl bromide, butyl chloride, methyl alcohol, ethyl alcohol, dimethyl sulfate and diethyl sulfate.

The process of this invention is carried out by submitting the silicon and aluminum compounds, the starting compounds, to a hydrothermal synthetic reaction in an aqueous medium in the presence of a fluorine compound, an organic cation and an alkali. The crystallization by means of the hydrothermal synthetic reaction is carried out at a temperature ranging between 100° and 200°C, preferably 130—170°C. It is preferred to perform the reaction under autogenous pressure. For obtaining the invention crystals possessing a unique crystal form, one of the important requisites is the control of the temperature conditions. As to the rate of temperature rise in crystallizing the starting mixture prepared at room temperature, the faster, the better. As a practical matter, the preferred rate of temperature rise is at least 1°C per minute, most preferred being a value of at least 2°C per minute. If the rate of temperature rise is less than 1°C per minute, the time required for raising the temperature of the starting material, which is at room temperature, to the crystallization temperature becomes prolonged, and hence the time that the mixture is held in a temperature zone not optimal for the crystallization becomes long. As a consequence, the growth of other classes of crystal forms or formation of seed crystals takes precedence during this period, and it becomes impossible to achieve an adequate growth of the desired crystals.

The time required for crystallization varies depending upon such factors as the crystallization temperature and the mole ratio of the starting materials compounded, but it is usually within the range 4—150 hours. For example, when the crystallization is carried out at 150°C, crystals of desired size can be obtained in 20—100 hours. While agitation at the time of crystallization conduces to desirable effects when the time required for the crystallization is short, the effects are small when the crystallization time extends over 20 hours.

A desirable practice is to perform the shear mixing of the starting mixture to be crystallized prior to submitting it to the hydrothermal synthetic reaction, since it becomes possible to achieve a thorough mixing of the starting mixture. By shear mixing is meant a mixing action which sets up a shearing stress in the starting mixture. For example, this can be achieved by the use of, say, a high-speed rotary mixer, a homogenizer or a colloid mill. When a mixer of this type is used, a shearing stress can be set up in the starting mixture usually by rotating the rotary member such that its terminal edge portion has a linear speed of at least 10 meters per second.

In practicing the process of this invention, it has been found that desirable results can be had in producing the crystals of this invention by using the various compounds that are used in the reaction in the ranges indicated below.

| | Usual range (mole ratio) | Preferred range (mole ratio) | Most preferred range (mole ratio) |
|---|---|---|---|
| $SiO_2/Al_2O_3$ | At least 50 | 100—3000 | 200—2000 |
| $R_4N^+/SiO_2$ | At least 0.1 | 0.1—1.0 | 0.2—0.8 |
| $Na^+/SiO_2$ | 0.1—0.8 | 0.15—0.7 | 0.2—0.6 |
| $F^-/SiO_2$ | At least 0.1 | 0.1—1.0 | 0.2—0.8 |
| $H_2O/SiO_2$ | At least 10 | 10—100 | 30—70 |

In the above table the silicon compound has been shown as $SiO_2$, while the aluminum compound has been shown as $Al_2O_3$. On the other hand, $R_4N^+$, $Na^+$ and $F^-$ represent respectively an organic cation, alkali and a fluorine compound.

As regards the $SiO_2/Al_2O_3$ mole ratio of the starting mixture, this mole ratio becomes directly the $SiO_2/Al_2O_3$ mole ratio in the crystal in almost all instances. If the $SiO_2/Al_2O_3$ mole ratio is less than 50, the $SiO_2/Al_2O_3$ mole ratio of the crystal becomes too small. The readily available commercial silica sol usually contains about 1000 parts per million of $Al_2O_3$. Hence, it is also possible to obtain the crystals of this invention by practicing the invention seemingly without adding especially an aluminum compound but by using only this silica sol. If the $R_4N^+/SiO_2$ mole ratio is less than 0.1, difficulty will be experienced in forming the crystals in this invention with good reproducibility.

Further, the $Na^+/SiO_2$ mole ratio is also important. If this mole ratio is less than 0.1, the crystallization becomes inadequate, and only a gelled mixture can be obtained. On the other hand, if the $Na^+/SiO_2$ mole ratio exceeds 0.8, the crystals become small.

If the $F^-/SiO_2$ mole ratio is less than 0.1, the effects obtained by the addition of fluorine do not appear readily, and hence crystals having a unique crystal form cannot be obtained. As the $F^-/SiO_2$ mole ratio becomes greater, there is a tendency to an increase in the ratio of the longest edge to the shortest edge.

On the other hand, when the $H_2O/SiO_2$ mole ratio becomes less than 10, the crystallization becomes inadequate.

Only when the foregoing mole ratios are all satisfied in compounding the materials does it become possible to prepare the crystals having a unique crystal form with good reproducibility.

In the case of the crystals of this invention their particles are large and the acid sites concealed. Hence, the crystals of this invention are suitably used directly as an adsorbent without any pretreatment. For example, they can be used conveniently for separation of the various hydrocarbon isomers or for the adsorption of organic substances from aqueous solutions of organic substances. Again, since the acid sites of the crystals of this invention become revealed by an acid treatment, they can be used as a solid acid catalyst. They are thus suitable for use as catalyst in the isomerization, disproportionation and alkylation of hydrocarbons. Further, they are also suitably used in the production of olefins, paraffin and aromatic compounds from methanol. The acid-treated crystals of this invention demonstrate especially good performances when used as catalyst for the synthesis of particularly the olefins from methanol and/or diethyl ether by the dehydration reaction in that their rate of catalytic degradation is small and that they give the intended products with a high selectivity.

The following examples will serve to more fully illustrate the present invention.

Example 1

To a solution in 185 g of water of 0.12 g of sodium aluminate (containing 34.9 wt% and 33.3 wt% of

$Al_2O_3$ and $Na_2O$, respectively), 1.5 g of caustic soda and 2.3 g of ammonium fluoride was added 14.3 g of tetrapropylammonium bromide, and the mixture was rendered into a homogeneous aqueous solution. After adding 28.5 g of 40 wt% silica gel to the foregoing aqueous solution, the mixture was placed in a mixer and mixed at 10,000 rpm (linear speed of the blade tip 45 meters per second) at room temperature for 3 minutes. The starting mixture was then transferred to a corrosion-resistant pressure vessel, after which the temperature was raised to 150°C over the course of one hour and then held in this state for 61 hours under autogenous pressure. This was followed by cooling the reaction product, separating the resulting crystals by filtration and washing the crystals in water until the wash liquid became neutral followed by drying the crystals at 110°C for 10 hours.

The mole ratios between the materials contained in the starting mixture, the crystallization conditions, the size of the crystals formed, the X-ray diffraction line data and the fluorine content of the crystals are shown in Table 1.

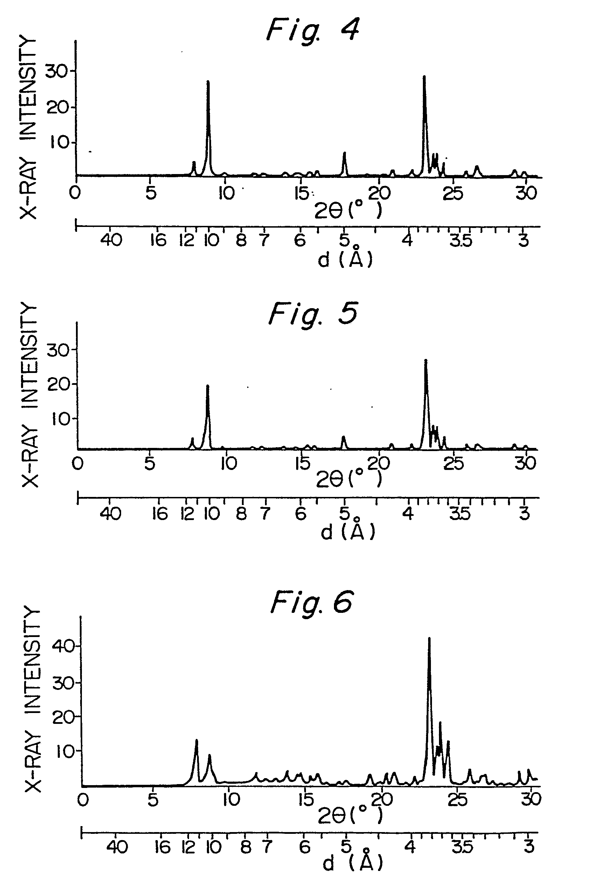

The photomicrograph and the X-ray diffraction pattern (standard powder method, $CuK_\alpha$) are shown in Figures 1 and 4, respectively. The minimum graduation of the scale in Figure 1 is 5.2 micrometers.

Examples 2—7

The experiment was operated as in Example 1 but varying the mole ratios between the starting materials, and the temperature and time of crystallization.

The conditions and the results obtained are shown together in Table 1.

The photomicrograph and X-ray diffraction pattern of the crystals obtained in Example 3 are shown in Figures 2 and 5, respectively. The minimum graduation of the scale in Figure 2 is 5.2 micrometers.

TABLE 1

| Example | 1 | 2 | 3 | 4 | 5 | 6 | 7 |
|---|---|---|---|---|---|---|---|
| Mole ratio between starting materials | | | | | | | |
| $SiO_2/Al_2O_3$ | 462 | 474 | 476 | 454 | 499 | 474 | 1856 |
| $Na^+/SiO_2$ | 0.22 | 0.22 | 0.22 | 0.22 | 0.62 | 0.22 | 0.64 |
| $R_4N^+/SiO_2$ | 0.28 | 0.28 | 0.55 | 0.28 | 0.55 | 0.28 | 0.56 |
| $F^-/SiO_2$ | 0.33 | 0.11 | 0.43 | 0.21 | 0.56 | 0.33 | 0.56 |
| $H_2O/SiO_2$ | 59.2 | 59.3 | 58.5 | 58.9 | 59.9 | 59.1 | 60.3 |
| Reaction temperature (°C) | 150 | 153 | 150 | 150 | 152 | 150 | 152 |
| Reaction time (hr) | 61 | 60 | 60 | 63 | 64 | 65 | 64 |
| Size of crystals*1 (μm) | 75×14 | 64×20 | 70×16 | 45×20 | 45×20 | 68×18 | 84×14 |
| X-ray diffraction line data of crystals (spacing)*2 | | | | | | | |
| 11.3 Å | 13 | 4 | 13 | 7 | 5 | 14 | 7 |
| 10.0 | 94 | 100 | 72 | 100 | 100 | 91 | 100 |
| 5.01 | 22 | 21 | 15 | 24 | 24 | 23 | 26 |
| 3.84 | 100 | 53 | 100 | 54 | 82 | 100 | 63 |
| 3.75 | 24 | 10 | 30 | 15 | 21 | 23 | 17 |
| 3.72 | 25 | 6 | 26 | 13 | 11 | 25 | 12 |
| 3.65 | 15 | 6 | 16 | 9 | 8 | 15 | 9 |
| Fluorine content of crystals (wt%) | 0.9 | 0.3 | 1.1 | 0.6 | 1.3 | 0.9 | 1.2 |

*1 Length of longest edge×length of shortest edge shown.

*2 Relative intensities at the spacings (unit Å) are shown. For example, in the case of the crystals of Example 1 the relatively intensity of the X-ray diffraction line corresponding to the spacing 3.84 Å is shown to be the maximum (100). Similarly, in the case of the crystals of Example 2, the relative intensity of the X-ray diffraction line corresponding to the spacing 10.0 Å is shown to be the maximum (100).

Comparative Examples 1—3

The experiments were operated as in Example 1, except that the fluorine compound was not added and the mole ratios between the starting materials and the temperature and time of crystallization were varied.

The results obtained are shown in Table 2.

The photomicrograph and X-ray diffraction pattern of the crystals obtained in Comparative Example 2 are shown in Figures 3 and 6, respectively. The minimum graduation of the scale in Figure 3 is 5.2 microns.

TABLE 2

| Comparative Example | 1 | 2 | 3 |
|---|---|---|---|
| Mole ratio between starting materials | | | |
| $SiO_2/Al_2O_3$ | 457 | 485 | 1105 |
| $Na^+/SiO_2$ | 0.15 | 0.62 | 0.62 |
| $R_4N^+SiO_2$ | 0.27 | 0.54 | 0.54 |
| $H_2O/SiO_2$ | 60.2 | 59.7 | 59.8 |
| Reaction temperature (°C) | 150 | 153 | 148 |
| Reaction time (hr) | 135 | 135 | 135 |
| Size of crystals*[1] (μm) | 20×15 | 0.5×0.5 | 5×5 |
| X-ray diffraction line data of crystals (spacing)*[2] | | | |
| 11.3 Å | 22 | 30 | 34 |
| 10.0 | 27 | 20 | 21 |
| 5.01 | 4 | 3 | 4 |
| 3.84 | 100 | 100 | 100 |
| 3.75 | 29 | 27 | 26 |
| 3.72 | 41 | 43 | 48 |
| 3.65 | 27 | 30 | 32 |

*[1] and *[2] have the same meanings as in Table 1.

Reference Example

This reference example is given for illustrating the preparation of olefins from methanol by the dehydration reaction using a solid acid catalyst prepared from the new crystalline aluminosilicate of this invention.

(1) The crystalline aluminosilicate powder obtained by the procedure described in Example 2 was calcined in the air at 550°C for 5 hours, after which the calcined product was treated with a 18 wt% aqueous hydrochloric acid solution at 90°C for 5 hours followed by cooling, separation by filtration and washing in water until no chlorine ions could be detected in the wash water and thereafter dried at 110°C for 10 hours. After adding 20 wt% silica sol as carrier to the thus obtained $H^+$ type crystalline aluminosilicate powder and thorough mixing of the components, the mixture was molded using a perforated plate having a diameter of 3 mm and a depth of 3 mm. After the molded product was dried, it was removed from the perforated plate, calcined in the air at 550°C for 5 hours to prepare the catalyst. This catalyst contained 80 wt% of the aluminosilicate and 20 wt% of silica as carrier.

(2) While maintaining at 350°C a catalyst bed of the solid acid catalyst prepared as described hereinabove, methanol was passed through using water as a diluent (methanol partial pressure 0.35 atm, methanol feeding speed 3.4 $hr^{-1}$ (LHSV)).

On analysis of the resulting reaction mixture, it was found that the conversion was 93%, the selectivity for hydrocarbons 42 wt%, the selectivity for dimethyl ether 3 wt% and the selectivity for water 55 wt%. As a consequence, the composition of the resulting hydrocarbons was as follows:

| Constituent | Wt% |
|---|---|
| Ethylene | 21 |
| Propylene | 19 |
| Butene | 10 |
| Methane | 0.3 |
| Ethane | 0.1 |
| Propane | 2.3 |
| Butane | 4.6 |
| Pentane | 5.7 |
| $C_5$≦olefins, $C_6$≦paraffins and aromatics | 37 |
| (p-Xylene content of $C_8$ aromatic | 93) |

**Claims**

1. A crystalline aluminosilicate characterized by having in its X-ray diffraction pattern determined by the standard powder method using Kα radiation of copper at least seven diffraction lines showing the following relative intensities at the positions of the following respective diffraction angles (2θ):

| Diffraction angle (2θ, deg) | Relative intensity |
|---|---|
| 7.9±0.3 | not more than 30 |
| 8.8±0.3 | 30—100 |
| 17.7±0.3 | 5—40 |
| 23.2±0.3 | 30—100 |
| 23.7±0.3 | 5—40 |
| 23.9±0.3 | not more than 40 |
| 24.4±0.3 | not more than 30, |

taking the intensity of the diffraction line at a diffraction angle of 8.8°±0.3° or 23.2°±0.3° as 100 and characterized in that the ratio of the length of the longest direction of the crystals thereof to that of the shortest direction of said crystals which crosses the longest direction at right angles thereto is 1.5 or higher and the length of the shortest direction is at least 14 micrometers.

2. A crystalline aluminosilicate according to claim 1 characterized in that it contains fluorine.

3. A crystalline aluminosilicate according to claim 1 or 2 characterized in that its crystalline form is basically a pillar-like octahedron.

4. A crystalline aluminosilicate according to any one of the preceding claims characterized in that its crystalline form is composed basically of two opposing surfaces in the shape of an elongate hexagon and six tetragonal surfaces.

5. A crystalline aluminosilicate according to any one of the preceding claims characterized in that the length of the longest direction is at least 45 micrometers.

6. A crystalline aluminosilicate according to any one of the preceding claims characterized in that the $SiO_2/Al_2O_3$ mole ratio is at least 50.

7. A crystalline aluminosilicate according to claim 6 characterized in that the $SiO_2/Al_2O_3$ mole ratio is from 100 to 3000.

8. A crystalline aluminosilicate according to claim 6 characterized in that the $SiO_2/Al_2O_3$ mole ratio is from 200 to 2000.

9. A process for producing a crystalline aluminosilicate as claimed in any one of the preceding claims

containing fluorine by submitting a silicon compound and an aluminium compound to a hydrothermal reaction in an aqueous medium in the presence of an organic cation and an alkali, characterized in that said hydrothermal reaction is carried out in the presence of a fluorine compound that forms fluoride ions during the hydrothermal reaction.

10. A process according to claim 9 characterized in that said fluorine compound is a compound that dissociates to form fluoride ions when dissolving in water.

11. A process according to claim 10 characterized in that said fluorine compound is sodium fluoride or ammonium fluoride.

12. A process according to claim 9 characterized in that said fluorine compound is a compound that forms fluoride ions by hydrolysis.

13. A process according to claim 12 characterized in that said fluorine compound is silicon fluoride, boron fluoride or sulfur fluoride.

14. A process according to any one of claims 9 to 13 characterized in that said hydrothermal reaction is effected at a temperature of from 100° to 200°C.

15. A process according to any one of claims 9 to 14 characterized in that said organic cation is present in such an amount that the organic cation/$SiO_2$ mole ratio is at least 0.1.

16. A process according to any one of claims 9 to 15 characterized in that said alkali is present in such an amount that the $Na^+/SiO_2$ mole ratio is at least 0.1.

17. A process according to any one of claims 9 to 16 characterized in that said fluorine compound is present in such an amount that the $F^-/SiO_2$ mole ratio is at least 0.1.

18. A process according to any one of claims 9 to 17 characterized in that water is used in such an amount that the $H_2O/SiO_2$ mole ratio is at least 10.

19. A process according to any one of claims 9 to 18 characterized in that the temperature of the reaction system is raised to the desired temperature for practicing the hydrothermal reaction at a rate of at least 1°C per minute.

20. A process according to any one of claims 9 to 19 characterized in that the fluorine-containing crystalline aluminosilicate is further treated to reveal the acid-sites of the crystals.

21. Use of a crystalline aluminosilicate as claimed in any one of claims 1 to 8 which has been produced by a process as claimed in any one of claims 9 to 20 as an absorbent or as a solid acid catalyst.

**Revendications**

1. Un aluminosilicate cristallisé caractérisé par le fiat que son spectre de diffraction de rayons X, établi par la méthode normale sur poudre avec la radiation Kα du cuivre, présente au moins sept raies de diffraction avec les intensités relatives suivantes aux positions des angles de diffraction respectifs indiqués (2θ):

| Angle de diffraction (2θ, deg) | Intensité relative |
|---|---|
| 7,9±0,3 | ne dépasse pas 30 |
| 8,8±0,3 | 30—100 |
| 17,7±0,3 | 5—40 |
| 23,2±0,3 | 30—100 |
| 23,7±0,3 | 5—40 |
| 23,9±0,3 | ne dépasse pas 40 |
| 24,4±0,3 | ne dépasse pas 30, |

en prenant comme étant égale à 100 l'intensité de la raie de diffraction à un angle de 8,8°±0,3° ou de 23,2°±0,3°, et il se caractérise aussi en ce que le rapport de la longueur de la plus longue direction des cristaux à celle de leur plus courte direction orthogonale avec la plus longue est de 1,5 ou plus, et la longueur de la plus courte direction est d'au moins 14 micromètres.

2. Aluminosilicate cristallisé selon la revendication 1, caractérisé en ce qu'il contient du fluor.

3. Aluminosilicate cristallisé selon la revendication 1 ou 2, caractérisé en ce que sa forme cristalline est essentiellement un octaèdre en forme de colonne.

4. Aminosilicate cristallisé selon l'une quelconque des revendications précédentes, caractérisé en ce que sa forme cristalline comprend essentiellement deux surfaces opposées ayant la forme d'un hexagone allongé et six surfaces tétragonales.

5. Aluminosilicate cristallisé selon l'une quelconque des revendications précédentes, caractérisé en ce que la longueur de la plus longue direction est d'au moins 45 micromètres.

6. Aluminosilicate cristallisé selon l'une quelconque des revendications précédentes, caractérisé en ce que le rapport molaire $SiO_2/Al_2O_3$ est d'au moins 50.

7. Aluminosilicate cristallisé selon la revendication 6, caractérisé en ce que le rapport molaire $SiO_2/Al_2O_3$ est de 100 à 3000.

8. Aluminosilicate cristallisé selon la revendication 6, caractérisé en ce que le rapport molaire $SiO_2/Al_2O_3$ est de 200 à 2000.

9. Un procédé de production d'aluminosilicates cristallisés selon l'une quelconque des revendications précédentes, contenant du fluor, selon lequel on soumet un composé du silicium et un composé d'aluminium à une réaction hydrothermique dans un milieu aqueux en présence d'un cation organique et d'un alcali, procédé caractérisé en ce que la réaction est effectuée également en présence d'un composé du fluor qui forme des ions fluorure au cours de la réaction.

10. Procédé selon la revendication 9 caractérisé en ce que le composé du fluor est un composé qui se dissocie en donnant des ions fluorure par dissolution dans l'eau.

11. Procédé selon la revendication 10 caractérisé en ce que le composé du fluor est le fluorure de sodium ou d'ammonium.

12. Procédé selon la revendication 9, caractérisé en ce que le composé du fluor est un composé qui forme des ions fluorure par hydrolyse.

13. Procédé selon la revendication 12 charactérisé en ce que le composé du fluor es le fluorure de silicium, de bore ou de soufre.

14. Procédé selon l'une quelconque des revendications 9 à 13, caractérisé en ce que la réaction hydrothermique est effectuée à une température de 100 à 200°C.

15. Procédé selon l'une quelconque des revendications 9 à 14, caractérisé en ce que le cation organique est ajouté dans une proportion donnant un rapport molaire cation organique/$SiO_2$ d'au moins 0,1.

16. Procédé selon l'une quelconque des revendications 9 à 15, caractérisé en ce que l'alcali est ajouté dans une proportion donnant un rapport molaire $Na^+/SiO_2$ d'au moins 0,1.

17. Procédé selon l'une quelconque des revendications 9 à 16, caractérisé en ce que le composé du fluor est ajouté dans une proportion donnant un rapport molaire $F^-/SiO_2$ d'au moins 0,1.

18. Procédé selon l'une quelconque des revendications 9 à 17, caractérisé en ce que l'eau est dans une proportion donnant un rapport molaire $H_2O/SiO_2$ d'au moins 10.

19. Procédé selon l'une quelconque des revendications 9 à 18, caractérisé en ce qu'on élève la température du mélange réactionnel jusqu'à la température voulue pour la réaction à une vitesse d'au moins 1°C par minute.

20. Procédé selon l'une quelconque des revendications 9 à 19, caractérisé en ce que l'on traite ensuite l'aluminosilicate cristallisé contenant du fluor pour révéler les sites acides des cristaux.

21. L'emploi d'un aluminosilicate cristallisé selon l'une quelconque des revendications 1 à 8, qui a été obtenu par un procédé selon l'une quelconque des revendications 9 à 20, comme produit absorbant ou comme catalyseur acide solide.

**Patentansprüche**

1. Kristallines Aluminosilicat, dadurch gekennzeichnet, daß es im nach der Standardpulvermethode unter Verwendung der Kα-Strahlung von Kupfer bestimmten Röntgenbeugungsbild mindestens sieben Beugungslinien aufweist, welche die folgenden relativen Intensitäten bei den Positionen der folgenden jeweiligen Beugungswinkeln (2θ) aufweist:

| Beugungswinkel (2θ, Grad) | Relative Intensität |
|---|---|
| 7,9±0,3 | höchstens 30 |
| 8,8±0,3 | 30—100 |
| 17,7±0,3 | 5—40 |
| 23,2±0,3 | 30—100 |
| 23,7±0,3 | 5—40 |
| 23,9±0,3 | höchstens 40 |
| 24,4±0,3 | höchstens 30, |

wobei die Intensität der Beugungslinie bei einem Beugungswinkel von 8,8°±0,3° oder 23,2°±0,3° als 100 genommen wird, und weiter dadurch gekennzeichnet, daß das Verhältnis der länge der längsten Richtung seiner Kristalle zu derjenigen der kürzesten Richtung der genannten Kristalle, die die längste Richtung in

rechten Winkeln dazu kreuzt, 1,5 oder höher ist und daß die Länge der kürzesten Richtung mindestens 14 µm ist.

2. Kristallines Aluminosilicat nach Anspruch 1, dadurch gekennzeichnet, daß es Fluor enthält.

3. Kristallines Aluminosilicat nach Anspruch 1 oder 2, dadurch gekennzeichnet, daß seine kristalline Form dem Grunde nach ein säulenartiges Octahedron ist.

4. Kristallines Aluminosilicat nach einem der vorstehenden Ansprüche, dadurch gekennzeichnet, daß seine kristalline Form in wesentlichen aus zwei gegenüberliegenden Oberflächen in der Gestalt eines länglichen Hexagons und sechs tetragonalen Oberflächen zusammengesetzt ist.

5. Kristallines Aluminosilicat nach einem der vorstehenden Ansprüche, dadurch gekennzeichnet, daß die Länge der längsten Richtung mindestens 45 µm ist.

6. Kristallines Aluminosilicat nach einem der vorstehenden Ansprüche, dadurch gekennzeichnet, daß das $SiO_2/Al_2O_3$-Molverhältnis mindestens 50 ist.

7. Kristallines Aluminosilicat nach Ancpruch 6, dadurch gekennzeichnet, daß das $SiO_2/Al_2O_3$-Molverhältnis 100 bis 3000 ist.

8. Kristallines Aluminosilicat nach Ancpruch 6, dadurch gekennzeichnet, daß das $SiO_2/Al_2O_3$-Molverhältnis 200 bis 2000 ist.

9. Verfahren zur Herstellung eines kristallinen Aluminosilicats nach einem der vorstehenden Ansprüche, welches Fluor enthält, durch Unterwerfen einer Siliciumverbindung und einer Aluminiumverbindung einer hydrothermischen Reaktion in einem wäßrigen Medium in Gegenwart eines organischen Kations und eines Alkalis, dadurch gekennzeichnet, daß man die genannte hydrothermische Reaktion in Gegenwart einer Fluorverbindung, die während der hydrothermischen Reaktion Fluoridionen bildet, durch führt.

10. Verfahren nach Anspruch 9, dadurch gekennzeichnet, daß die Fluorverbindung eine Verbindung ist, die beim Auflösen in Wasser unter Bildung von Fluoridionen dissoziiert.

11. Verfahren nach Anspruch 10, dadurch gekennzeichnet, daß die Fluorverbindung Natriumfluorid oder Ammoniumfluorid ist.

12. Verfahren nach Anspruch 9, dadurch gekennzeichnet, daß die Fluorverbindung eine Verbindung ist, die durch Hydrolyse Fluoridionen bildet.

13. Verfahren nach Anspruch 12, dadurch gekennzeichnet, daß die Fluorverbindung Siliciumfluorid, Borfluorid oder Schwefelfluorid ist.

14. Verfahren nach einem der Ansprüche 9 bis 13, dadurch gekennzeichnet, daß man die hydrothermische Reaktion bei einer Temperatur von 100 bis 200°C durchführt.

15. Verfahren nach einem der Ansprüche 9 bis 14, dadurch gekennzeichnet, daß das organische Kation in einer solchen Menge vorhanden ist, daß das Molverhältnis von organischem Kation zu $SiO_2$ mindestens 0,1 beträgt.

16. Verfahren nach einem der Ansprüche 9 bis 15, dadurch gekennzeichnet, daß das Alkali in einer solchen Menge vorhanden ist, daß das $Na^+/SiO_2$-Molverhältnis mindestens 0,1 beträgt.

17. Verfahren nach einem der Ansprüche 9 bis 16, dadurch gekennzeichnet, daß die Fluorverbindung in einer solchen Menge vorhanden ist, daß das $F^-/SiO_2$-Molverhältnis mindestens 0,1 beträgt.

18. Verfahren nach einem der Ansprüche 9 bis 17, dadurch gekennzeichnet, daß man das Wasser in einer solchen Menge verwendet, daß das $H_2O/SiO_2$-Molverhältnis mindestens 10 beträgt.

19. Verfahren nach einem der Ansprüche 9 bis 18, dadurch gekennzeichnet, daß die Temperatur des Reaktionssystems auf die gewünschte Temperatur zur Durchführung der hydrothermischen Reaktion mit einer Geschwindigkeit von mindestens 1°C pro Minute erhöht wird.

20. Verfahren nach einem der Ansprüche 9 bis 19, dadurch gekennzeichnet, daß man das Fluor enthaltende kristalline Aluminosilicat weiter behandelt, um die Säurestellen der Kristalle aufzudecken.

21. Verwendung eines kristallinen Aluminosilicats nach einem der Ansprüche 1 bis 8, das nach einem Verfahren nach einem der Ansprüche 9 bis 20 hergestellt worden ist, als Absorbens oder fester saurer Katalysator.

Fig. 1

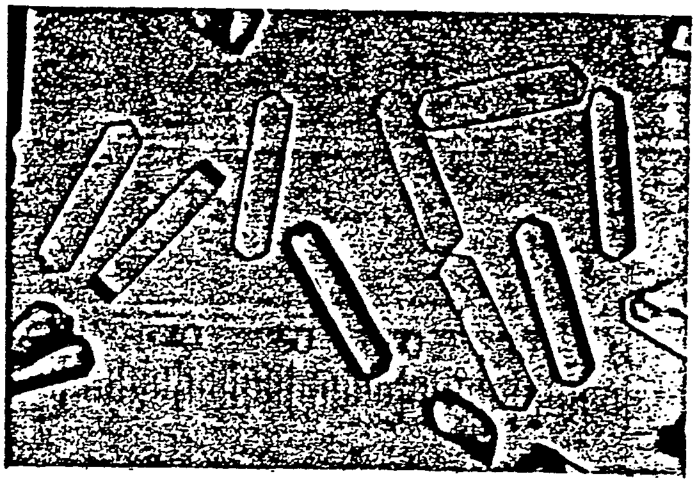

Fig. 2

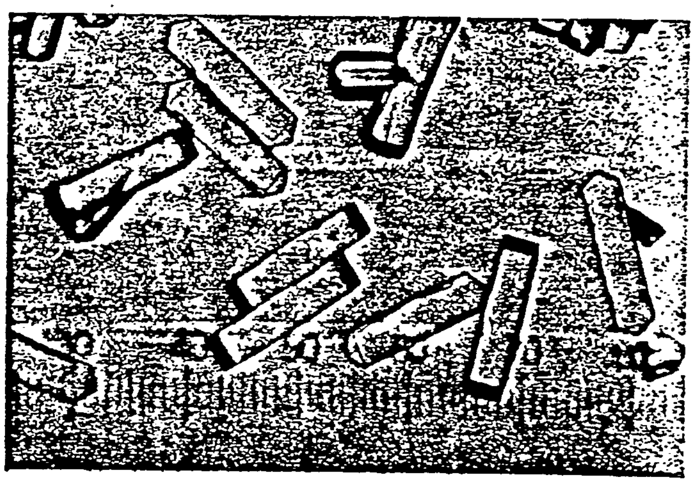

Fig. 3

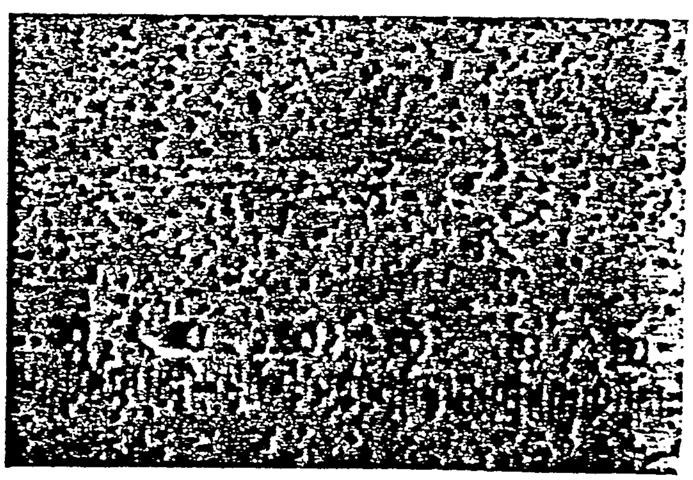

Fig. 4

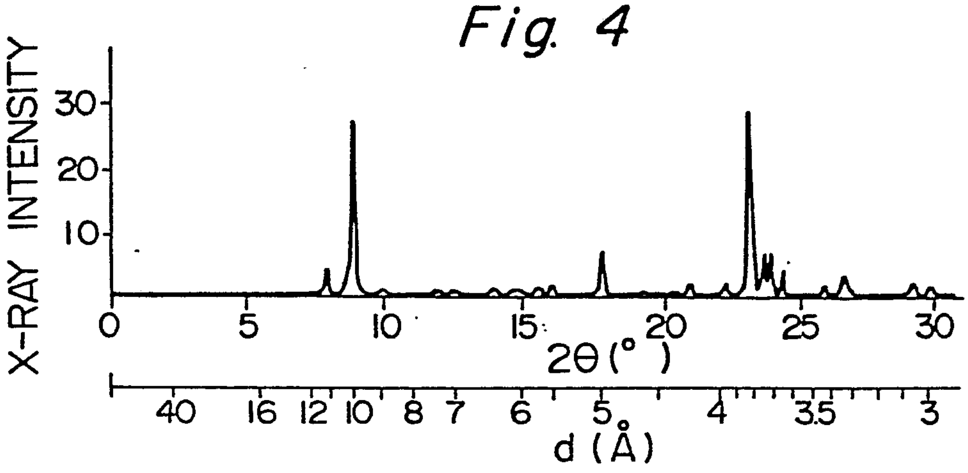


Fig. 5

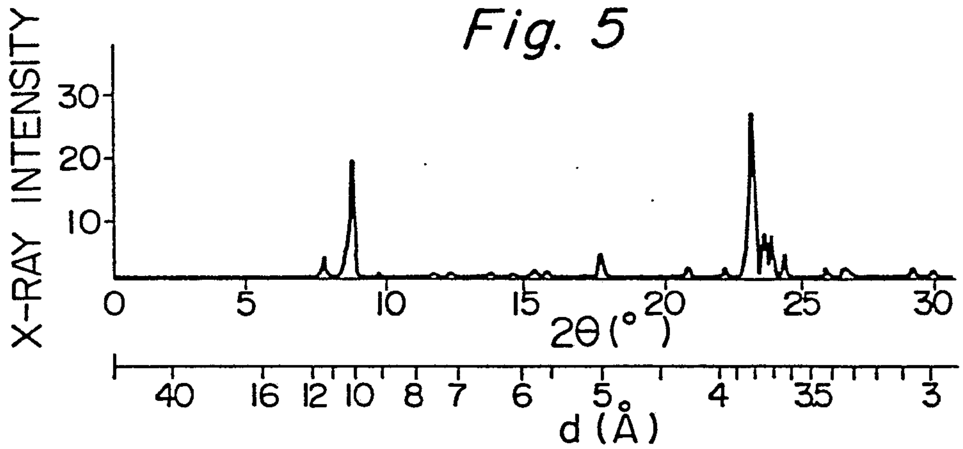


Fig. 6